# EUROPEAN PATENT APPLICATION

(11) **EP 3 855 426 A1**
(43) Date of publication of application: **28.07.2021**
(21) Application number: 21151509.3
(22) Date of filing: 14.01.2021
(51) Int. Cl.: G10K 11/178, B23K 9/00

(54) **SYSTEMS AND METHODS TO REDUCE PERCEIVED AUDIBLE WELDING NOISE**

(30) Priority: 21.01.2020 US 202016748369
(71) Applicant: Illinois Tool Works INC., Glenview, IL 60025 (US)
(72) Inventor: SCHNEIDER, Joseph C., Illinois, 60025 (US)
(74) Representative: HGF

(57) **Abstract**

An example audio device includes: communication circuitry configured to receive a first signal representative of an audible sound associated with a welding-type waveform; audio processing circuitry configured to generate, based on the first signal, a welding noise cancellation signal to produce destructive interference to the audible sound; and a transducer configured to output the destructive interference using the welding noise cancellation signal.

## Description

### TECHNICAL FIELD

This disclosure relates generally to audible noise reduction in welding systems and, more particularly, to systems and methods to reduce perceived audible welding noise.

### BACKGROUND

Excessive noise, such as noise created by certain welding arc waveforms, could potentially distract the welder and diminish the level of the welder's concentration on the weld. Excessive noise could also lead to weld operator fatigue and/or other undesirable physical effects.

During the welding process, an electric arc may form between the welding torch and the workpiece. When using certain types of welding processes, such as AC and/or DC pulse waveforms, the electric arc may be a source of undesirable and consistent noise that emanates from the arc in the form of acoustic noise sound waves.

### SUMMARY

The present disclosure is directed to systems and methods for reducing perceived audible welding noise.

These and other advantages, aspects and novel features of the present disclosure, as well as details of an illustrated example thereof, will be more fully understood from the following description and drawings.

### BRIEF DESCRIPTION OF THE DRAWINGS

FIG. 1 illustrates an example welding system that reduces welding noise perceived by a weld operator during a welding process, in accordance with aspects of this disclosure.
FIG. 2 is a block diagram of an example welding system including a welding-type power supply configured to output welding-type power, in accordance with aspects of this disclosure.
FIG. 3 illustrates an example welding helmet equipped with an example noise cancellation audio device and which may be used to implement the helmet of FIG. 1, in accordance with aspects of this disclosure.
FIG. 4 is a more detailed block diagram of an example noise cancellation audio device that may be used to implement the audio devices of FIGS. 1 and/or 3.
FIG. 5 illustrates example welding headphones that include a noise cancellation audio device, in accordance with aspects of this disclosure.
FIG. 6 illustrates an example welding speaker system that includes a noise cancellation audio device, in accordance with aspects of this disclosure.
FIG. 7 illustrates an example instance of welding arc noise cancellation by destructive interference of two sound waves.
FIG. 8A illustrates the variations of the magnitude of an example welding arc noise sound wave with respect to time.
FIG. 8B illustrates the variations of the magnitude of an example welding arc destructive interference sound waves with respect to time.
FIG. 8C illustrates the variations of the magnitude of an example combined resulting acoustic wave with respect to time as perceived by a weld operator.
FIG. 9 illustrates an example of operation of the noise cancellation audio device(s) of FIGS. 1, 3, 4, 5, and/or 6, in accordance with aspects of this disclosure.
FIG. 10 is a flowchart representative of example machine-readable instructions which may be executed by disclosed noise cancellation audio device(s) to generate and output a welding noise cancellation signal, in accordance with aspects of this disclosure.
FIG. 11 is a flowchart representative of example machine-readable instructions which may be executed by disclosed power supplies and/or current sensors to transmit a signal representative of a current of a welding-type waveform, in accordance with aspects of this disclosure.
FIG. 12 is a flowchart representative of example machine-readable instructions which may be executed by disclosed noise cancellation audio device(s) to generate and output a welding noise cancellation signal, in accordance with aspects of this disclosure.
FIG. 13 is a flowchart representative of example machine-readable instructions which may be executed by disclosed power supplies to transmit a signal based on an audible sound waveform, in accordance with aspects of this disclosure.

The figures are not necessarily to scale. Where appropriate, similar or identical reference numbers are used to refer to similar or identical components.

### DETAILED DESCRIPTION

Conventional noise cancellation systems typically rely on direct measurements of the magnitude of ambient acoustic sound waves, and using the measurements to generate acoustic sound waves that are 180 degrees out of phase with the ambient sound waves. The resulting destructive interference between the original acoustic sound waves and the generated noise cancellation sound waves results in an acoustic sound wave at the listener's ear that is of lower magnitude than the original noise sound wave. As a result, the sound that is perceived by the listener is of a lower magnitude than the ambient sound.

In disclosed example systems and methods, the noise cancellation signal is not based on the measurement of the source noise sound waves, or not solely based on such source noise sound waves. Instead, disclosed example systems and methods generate and output noise cancellation signals based on properties and/or measurements of the electric current that is generated by the power supply for the purpose of generating the electric arc between the welding torch and the workpiece.

As used herein, a control circuit may include digital and/or analog circuitry, discrete and/or integrated circuitry, microprocessors, DSPs, etc., software, hardware and/or firmware, located on one or more boards, that form part or all of a controller, and/or are used to control a welding process, and/or a device such as a power source or wire feeder.

As used herein, the term "processor" means processing devices, apparatuses, programs, circuits, components, systems, and subsystems, whether implemented in hardware, tangibly embodied software, or both, and whether or not it is programmable. The term "processor" as used herein includes, but is not limited to, one or more computing devices, hardwired circuits, signal-modifying devices and systems, devices and machines for controlling systems, central processing units, programmable devices and systems, field-programmable gate arrays, application-specific integrated circuits, systems on a chip, systems comprising discrete elements and/or circuits, state machines, virtual machines, data processors, processing facilities, and combinations of any of the foregoing. The processor may be, for example, any type of general-purpose microprocessor or microcontroller, a digital signal processing (DSP) processor, an application-specific integrated circuit (ASIC). The processor may be coupled to, and/or integrated with a memory device.

As used, herein, the term "memory" and/or "memory device" means computer hardware or circuitry to store information for use by a processor and/or other digital device. The memory and/or memory device can be any suitable type of computer memory or any other type of electronic storage medium, such as, for example, read-only memory (ROM), random access memory (RAM), cache memory, compact disc read-only memory (CDROM), electro-optical memory, magneto-optical memory, programmable read-only memory (PROM), erasable programmable read-only memory (EPROM), electrically-erasable programmable read-only memory (EEPROM), a computer-readable medium, or the like.

As used herein, welding-type power refers to power suitable for welding, cladding, brazing, plasma cutting, induction heating, CAC-A and/or hot wire welding/preheating (including laser welding and laser cladding), carbon arc cutting or gouging, and/or resistive preheating.

As used herein, a welding-type power supply refers to any device capable of, when power is applied thereto, supplying suitable power for welding, cladding, brazing, plasma cutting, induction heating, laser (including laser welding, laser hybrid, and laser cladding), carbon arc cutting or gouging and/or resistive preheating, including but not limited to transformer-rectifiers, inverters, converters, resonant power supplies, quasi-resonant power supplies, switch-mode power supplies, etc., as well as control circuitry and other ancillary circuitry associated therewith.

Disclosed example audio devices include communication circuitry configured to receive a first signal representative of an audible sound associated with a welding-type waveform, audio processing circuitry configured to generate, based on the first signal, a welding noise cancellation signal to produce destructive interference to the audible sound, a transducer configured to output the destructive interference using the welding noise cancellation signal.

In some example audio devices, the communication circuitry is configured to receive a synchronization signal, and the audio processing circuitry is configured to synchronize the noise cancellation signal based on the synchronization signal. In some examples, the first signal identifies a frequency of the welding-type waveform. In some examples, the first signal identifies a magnitude of the welding-type waveform. Some example audio devices further include a microphone configured to receive the audible sound, in which the audio processing circuitry is configured to synchronize the welding noise cancellation signal based on the audible sound.

In some example audio devices, the audio device includes at least one of headphones, a welding helmet, or a loudspeaker. In some examples, the first signal is an analog signal representative of the welding-type waveform. In some examples, the first signal includes an identifier of a characteristic of the welding-type waveform, and the audio processing circuitry is configured to determine the welding noise cancellation signal based on the characteristic. In some examples, the audio processing circuitry is configured to determine the welding noise cancellation signal based on the characteristic using at least one of a lookup table or a function. In some examples, the welding-type waveform comprises at least one of an AC waveform or a DC pulse waveform.

Disclosed example welding apparatus include a current sensor configured to measure a current of a welding-type waveform and a transmitter configured to transmit a first signal representative of an audible sound associated with the welding-type waveform.

In some examples, the first signal identifies at least one of a frequency of the welding-type waveform or a magnitude of the welding-type waveform. In some examples, the first signal includes an analog waveform based on the measurement by the current sensor. In some examples, the current sensor includes at least one of a current transformer, Hall Effect sensor, a shunt current sensor, or a Rogowski coil configured to be coupled to a welding circuit transmitting the welding-type waveform.

Additional disclosed example welding apparatus include a current sensor configured to measure a current of a welding-type waveform, audio processing circuitry configured to generate, based on the current, a welding noise cancellation signal to produce destructive interference to the audible sound, and a transducer configured to output the destructive interference using the welding noise cancellation signal.

Disclosed example welding-type power supplies include power conversion circuitry configured to convert input power to welding-type power having a welding-type waveform, and a transmitter configured to transmit a first signal based on an audible sound associated with the welding-type waveform.

Some example welding-type power supplies further include control circuitry configured to control the power conversion circuitry to output the welding-type power and generate the first signal based on the welding-type waveform. In some examples, the first signal identifies at least one of a frequency of the welding-type waveform or a magnitude of the welding-type waveform. In some examples, the first signal comprises an analog waveform representative of the welding-type waveform. In some example power supplies, the first signal includes an audio signal representative of destructive interference to the audible sound associated with the welding-type waveform.

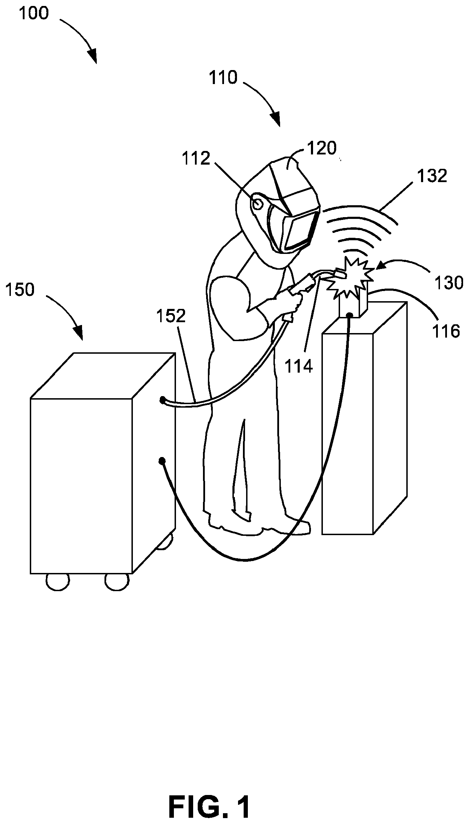

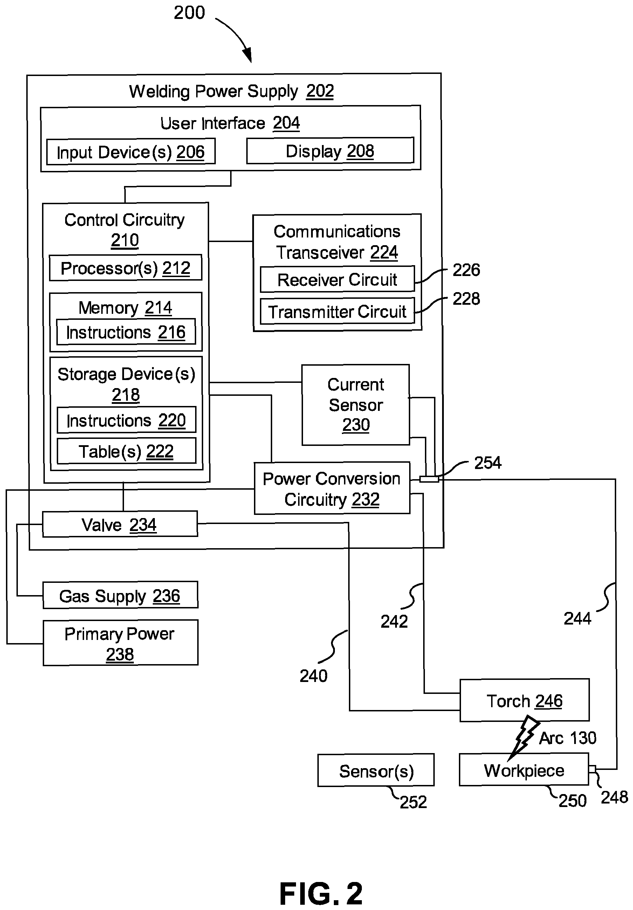

FIG. 1 illustrates an example welding system 100 that reduces welding noise perceived by a weld operator 110 during a welding process. The example weld operator 110 wears a welding helmet 120. The welding system 100 includes a welding-type power supply 150 and a welding torch 114. The welding torch 114 is connected to the welding-type power supply 150 via a weld cable 152. A more detailed discussion of the welding-type power supply 150 is provided below with reference to FIG. 2. The weld operator 110 uses the welding torch 114 to weld the welding workpiece 116. During the welding process, an electric arc 130 may form between the welding torch 114 and the workpiece 116. When using certain types of welding processes, such as AC waveforms or AC and/or DC pulse waveforms, the electric arc 130 may be a source of undesirable acoustic noise waves 132 that emanate from the arc 130.

The example welding helmet 120 may include a noise cancellation audio device 112. Additionally, or alternatively, the noise cancellation audio device 112 may be implemented in the welding system 100 using other audio devices, such as headphones and/or speakers. The noise cancellation audio device 112 may receive electric current signals, arc current and/or frequency data, and/or other information representative of the current waveform output at the welding arc 130 and/or representative of audible noise generated by the welding arc.

The example noise cancellation audio device 112 receives the current and/or noise signals and, based on the signals, generates destructive interference sound waves. The noise cancellation audio device 112 plays or outputs the destructive interference sound waves to the weld operator 110 to cancel the acoustic noise waves 132 and reduce the perceived volume of the audible welding noise to the weld operator 110.

FIG. 2 is a block diagram of an example welding system 200 having a welding-type power supply 202 and a welding torch 246. The welding system 200 powers, controls, and/or supplies consumables to a welding application. In the example of FIG. 2, the power supply 202, which may implement the power supply 150 of FIG. 1, supplies welding-type output power to the welding torch 246. The example welding torch 246 is configured for gas tungsten arc welding (GTAW), which may be used to perform welding processes involving DC welding-type current, pulsed DC welding-type current waveforms, and/or AC waveforms. Example pulse waveforms that may be output by the power supply 202 have a peak phase at a peak current and a background phase at a background current, and one pulse cycle includes one peak phase and one background phase.

The power supply 202 receives primary power 238 (e.g., from the AC power grid, an engine/generator set, a battery, or other energy generating or storage devices, or a combination thereof), conditions the primary power, and provides an output power to one or more welding devices in accordance with demands of the system 200. The primary power 238 may be supplied from an offsite location (e.g., the primary power may originate from the power grid). The power supply 202 includes power conversion circuitry 232, which may include transformers, rectifiers, switches, and so forth, capable of converting the AC input power to AC and/or DC output power as dictated by the demands of the system 200 (e.g., particular welding processes and regimes). The power conversion circuitry 232 converts input power (e.g., the primary power 238) to welding-type power based on a target amperage (e.g., a weld current setpoint) and outputs the welding-type power via a weld circuit.

The power supply 202 includes control circuitry 210 to control the operation of the power supply 202. The power supply 202 also includes a user interface 204. The control circuitry 210 receives input from the user interface 204, through which a user may choose a process and/or input desired parameters (e.g., a voltage, a current, a frequency, pulse peak current time, a pulse peak current percentage, a pulse background current time, a pulse background current percentage, an AC waveform type, an AC balance, a weld circuit inductance, etc.). The user interface 204 may receive inputs using one or more input devices 206, such as via a keypad, keyboard, physical buttons, switches, knobs, a mouse, a keyboard, a keypad, a touch screen (e.g., software buttons), a voice activation system, a wireless device, etc. Furthermore, the control circuitry 210 controls operating parameters based on input by the user as well as based on other current operating parameters. Specifically, the user interface 204 may include a display 208 for presenting, showing, or indicating, information to an operator.

The control circuitry 210 includes at least one controller or processor 212 that controls the operations of the power supply 202. The control circuitry 210 receives and processes multiple inputs associated with the performance and demands of the system 200. The processor 212 may include one or more microprocessors, such as one or more "general-purpose" microprocessors, one or more special-purpose microprocessors and/or ASICS, and/or any other type of processing device. For example, the processor 212 may include one or more digital signal processors (DSPs).

The example control circuitry 210 includes one or more storage device(s) 218 and one or more memory device(s) 214. The storage device(s) 218 (e.g., nonvolatile storage) may include ROM, flash memory, a hard drive, and/or any other suitable optical, magnetic, and/or solid-state storage medium, and/or a combination thereof. The storage device 218 stores data (e.g., data corresponding to a welding application), instructions 220 (e.g., software or firmware to perform welding processes), and/or any other appropriate data such a tables 222.

The memory device 214 may include a volatile memory, such as random access memory (RAM), and/or a nonvolatile memory, such as read-only memory (ROM). The memory device 214 and/or the storage device(s) 218 may store a variety of information and may be used for various purposes. For example, the memory device 214 and/or the storage device(s) 218 may store processor executable instructions 220 (e.g., firmware or software) for the processor 212 to execute. In addition, one or more control regimes for various welding processes, along with associated settings and parameters, may be stored in the storage device 218 and/or memory device 214.

In some examples, the welding-type power supply 202 may include a communications transceiver 224 and the communications transceiver 224 may include a receiver circuit 226 and a transmitter circuit 228.

In some examples, a gas supply 236 provides shielding gases, such as argon, helium, carbon dioxide, and so forth, depending upon the welding application. The shielding gas flows to a valve 234, which controls the flow of gas, and if desired, may be selected to allow for modulating or regulating the amount of gas supplied to a welding application. The valve 234 may be opened, closed, or otherwise operated by the control circuitry 210 to enable, inhibit, or control gas flow (e.g., shielding gas) through the valve 234. Shielding gas exits the valve 234 and flows through a cable 240 (which in some implementations may be packaged with the welding power output) to the welding torch 246, which provides the shielding gas to the welding application. In some examples, the welding system 200 does not include the gas supply 236, the valve 234, and/or the cable 240.

The welding torch 246 (e.g. the torch 114 of FIG. 1) delivers the welding power and/or shielding gas for a welding application. The welding torch 246 is used to establish the welding arc 130 between the welding torch 246 and a workpiece 250. A welding cable 242 couples the torch 246 to the power conversion circuitry 232 to conduct current to the torch 246. A work cable 244 couples the workpiece 250 to the power supply 202 (e.g., to the power conversion circuitry 232) to provide a return path for the weld current (e.g., as part of the weld circuit). The example work cable 244 is attachable and/or detachable from the power supply 202 for ease of replacement of the work cable 244. The work cable 244 may be terminated with a clamp 248 (or another power connecting device), which couples the power supply 202 to the workpiece 250.

In some examples, one or more sensors 252 are included with or connected to the welding torch 246 to monitor one or more welding parameters (e.g., power, voltage, current, inductance, impedance, etc.) to inform the control circuitry 210 during the welding process.

The example power supply 202 of FIG. 2 may be configured to measure the output current from the power conversion circuitry 232. For example, the power supply 202 may include a current sensor 230 to measure the output current. The example current sensor 230 may include any type of current sensor and associated measurement circuitry, such as a current transformer 254, a Hall Effect sensor, a shunt current sensor, a Rogowski coil, and/or any other type of current sensor. The output current measurements may include measurements of amplitude and/or frequency. For example, the control circuitry 210 may perform a Fast Fourier Transform (FFT) and/or other analysis on the current measurements to determine characteristics of the output current, such as frequencies and/or amplitudes of the output current that represent creation of audible noise by the welding arc 130 corresponding to the measured current.

Additionally or alternatively, the control circuitry 210 may monitor the parameters and/or feedback values to the control loop used by the control circuitry 210 to control the power conversion circuitry 232. For example, the control circuitry 210 may monitor parameters such as frequency, pulses per second, peak current magnitude, background current magnitude, and/or other parameters, and/or variables, such as current error, measured output current (e.g., current magnitude), and/or other variables.

Based on the output current, the control circuitry 210 transmits one or more signals to the noise cancellation audio device 112, in which the one or more signals are representative of an audible noise output by the arc 130 generated by the output current from the power conversion circuitry. For example, the communications transceiver 224 may communicate with the example welding helmet 120 and/or the example noise cancellation audio device 112 of FIG. 1 to provide the signal(s) to the example welding helmet 120 and/or the example noise cancellation audio device 112. The one or more signals may be analog or digital.

In some other examples, the control circuitry 210 may process the signals representative of the audible noise to generate a corresponding noise cancellation signal, and transmit the noise cancellation signal to the example welding helmet 120 and/or the example noise cancellation audio device 112. In this manner, the processing load on the noise cancellation audio device 112 may be reduced.

Additionally or alternatively, the control circuitry 210 may transmit a synchronization signal to the noise cancellation audio device 112, in which the synchronization signal enables the noise cancellation audio device 112 to synchronize predetermined points in the audible noise to corresponding points in the noise cancellation signal. For example, the control circuitry 210 may output a synchronization signal corresponding to the peak magnitude of the output current in a waveform cycle. When received by the noise cancellation audio device 112, the noise cancellation audio device 112 may synchronize the output of the noise cancellation signal based on match the peak noise cancellation signal to the synchronization signal representative of the peak magnitude of the welding current.

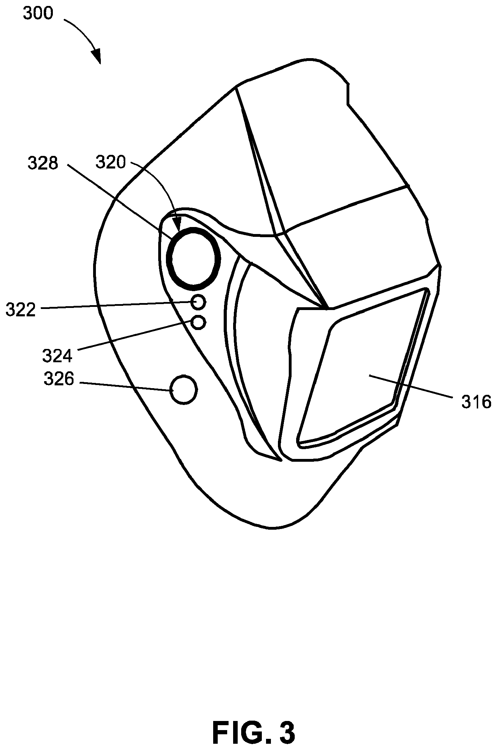

FIG. 3 illustrates an example welding helmet 300 equipped with an example noise cancellation audio device 320. The welding helmet 300 of FIG. 3 may be used to implement the welding helmet 120 that is depicted in FIG. 1.

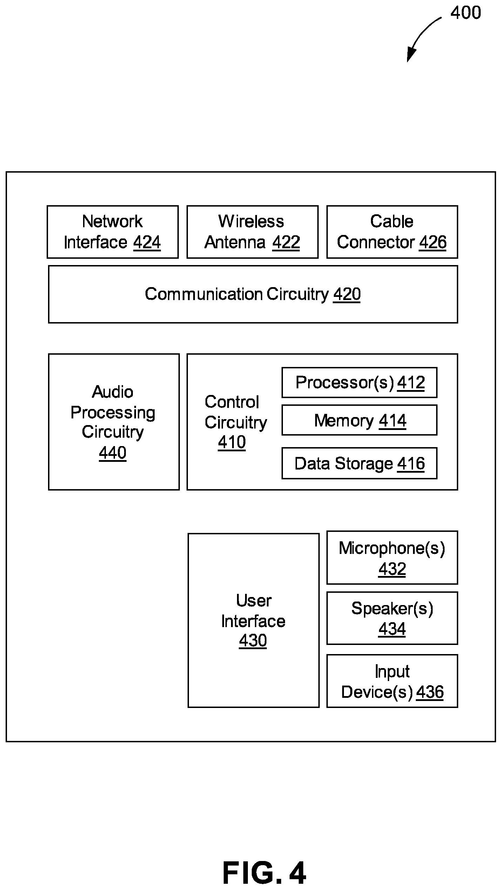

The example welding helmet 300 includes a lens 316, the noise cancellation audio device 320, and a phase adjustment knob 326. The example noise cancellation audio device 320 includes audio device communication circuitry 322, audio processing circuitry 324, and a transducer 328. An example implementation of the noise cancellation audio device 320 is described below with reference to FIG. 4.

The noise cancellation audio device 320 may be integrated into the welding helmet 300 and/or detachably coupled to the welding helmet 300. As described in more detail below, the noise cancellation audio device 320 receives (e.g., via the audio device communication circuitry 322) one or more signals representative of audible sound associated with (e.g., created by) a welding-type arc and/or welding-type waveform. In some examples, the received signals may be signals received from the power supply 150. In some other examples, the received signals are measured by a current sensor coupled to the weld circuit.

The noise cancellation audio device 320 generates (e.g., via the audio processing circuitry 324) a welding noise cancellation signal based on the received signal(s) to produce destructive interference to the audible sound. The transducer 328 outputs the destructive interference using the welding noise cancellation signal.

The wearer of the helmet (e.g., the weld operator 110 of FIG. 1) may adjust a phase of the welding noise cancellation signal output by the transducer 328 using the phase adjustment knob 326. By manually adjusting the phase, the wearer may correct for differences in distance between the transducer 328 and the wearer's ear, which may result in a phase shift between the preferred phase difference between the welding noise cancellation signal and the audible welding noise (e.g., 180 degrees). When combined with synchronization between the welding noise cancellation signal and the audible welding noise (e.g., using the times of peak magnitude), the phase adjustment knob 326 may maintain a relatively consistent noise cancellation for a given user.

FIG. 4 is a block diagram of noise cancellation audio device 400 that may be used to implement the noise cancellation audio devices 112, 320 of FIGS. 1 and/or 3. The example noise cancellation audio device 400 of FIG. 4 includes control circuitry 410, communication circuitry 420, a wireless antenna 422, a network interface 424, a cable connector 426, a user interface 430, one or more microphones 432, one or more speakers 434, one or more input device 436, and audio processing circuitry 440.

The example communication circuitry 420 (e.g., the audio device communication circuitry 322 of FIG. 3) may communicate with external devices via the wireless antenna 422, the network interface 424, and/or the cable connectors 426. For example, the communication circuitry 420 may communicatively couple the control circuitry 410 to the welding-type power supply 150, 202 (e.g., the communications transceiver 224).

The wireless antenna 422 may be any type of antenna suited for the frequencies, power levels, etc. used for radio frequency (RF) wireless communications (e.g., Wi-Fi, WiFi hotspot or MiFi, Bluetooth, Bluetooth Low Energy, Zigbee, NFC, cellular network, PAN/WPAN, BAN and/or the like) between the noise cancellation audio device 400 and other devices such as the welding-type power supply 150, 202, a wireless access point (WAP), other welding equipment, wireless base stations, phones, computers, etc. The example cable connector 426 may include, for example, an Ethernet port, a USB port, an HDMI port, a fiber-optic communications port, a FireWire port, a field bus port, a fiber optics port, and/or any other suitable port for interfacing with a wired or optical cable via which the noise cancellation audio device 400 may communicate with other devices such as welding equipment, wireless base stations, phones, computers, etc. Additionally or alternatively, the cable connector 426 may include sensor ports for receiving signals from a current sensor and/or other sensor(s).

The communication circuitry 420 interfaces the control circuitry 410 and/or the audio processing circuitry 440 to the antenna 422 and/or the cable connectors 426 for transmit and receive operations. For transmit operations, communication circuitry 420 receives data (e.g., the control circuitry 410), packetizes the data, and converts the data to physical layer signals in accordance with protocols in use by the communication circuitry 420. The data to be transmitted may include, for example, control signals for controlling the welding-type power supply 150,202. For receive operations, the communication circuitry 420 receives physical layer signals via antenna 422 and/or cable connectors 426, recovers data from the received physical layer signals (demodulate, decode, etc.), and provides the data to the control circuitry 410 and/or the audio processing circuitry 440. The received data may include, for example, signals representative of audible noise emanating from a welding arc, destructive interference signals, synchronization signals, and/or any other information. Example signals representative of the audible noise may include analog and/or digital data, one or more frequencies of the welding waveform, one or more current magnitudes of the welding waveform, samples of the current waveform, an FFT of the current waveform, and/or any other characteristics of the current waveform.

The control circuitry 410 controls the operation of the noise cancellation audio device 400. The example control circuitry 410 of FIG. 4 includes one or more processors 412, memory 414, and data storage 416. The processor(s) 412 may include one or more microprocessors, such as one or more "general-purpose" microprocessors, one or more special-purpose microprocessors and/or application specific integrated circuits (ASICS), or some combination thereof. For example, the processor(s) may include one or more reduced instruction set (RISC) processors (e.g., Advanced RISC Machine (ARM) processors), one or more digital signal processors (DSPs), and/or other appropriate processors. The processor(s) 412 may use data stored in the memory 414 and/or the data storage 416 to execute control processes.

The example memory 414 and/or the data storage 416 may store one or more lookup tables and/or functions, which may be accessed by the processor(s) 412 and/or the audio processing circuitry 440 to convert received information about the audible noise to destructive interference to at least partially cancel the audible noise and reduce the perceived volume of the audible noise to the wearer of the noise cancellation audio device 400. The data stored in the data storage 416 may be received via the operator interface, one or more input/output ports, a network connection, and/or be preloaded prior to assembly of the control circuitry 410.

The audio processing circuitry 440 may implement the audio processing circuitry 324 of FIG. 3. The audio processing circuitry 440 generates, based signals representative of audible sound, a welding noise cancellation signal to produce destructive interference to the audible sound. For example, the audio processing circuitry 440 may process signals, data, and information from the welding-type power supply 150, 202 (e.g. received via communication circuitry 420) that are representative of the welding current to generate audio signals that, when played, serve as destructive interference to the audio signals. The audio processing circuitry 440 outputs the destructive interference signals (e.g., to the weld operator 110) in the form of audio signals via the speaker(s) 434 or other audio transducers.

The speakers 434 may be integrated into, or attached to, a welding helmet (e.g., the welding helmet 300 of FIG. 3), worn by the operator on a headset separate from the welding helmet 300, and/or not worn by the operator (e.g., set on a surface near the operator or near the arc). The weld operator 110 may adjust the parameters of the noise cancellation audio device 400. For example, the weld operator 110 may use the control input devices 436 to adjust a phase of the destructive interference (e.g., via the phase adjustment knob 326), to adjust the volume of the speakers 434, and/or to set the parameters of the noise cancellation audio device 400 according to the personal characteristics and personal needs of the individual weld operator 110. In addition to adjusting the parameters of the noise cancellation audio device 400 via the input devices 436, the weld operator 110 may use voice commands, received through microphones 432, to adjust parameters of the noise cancellation audio device 400.

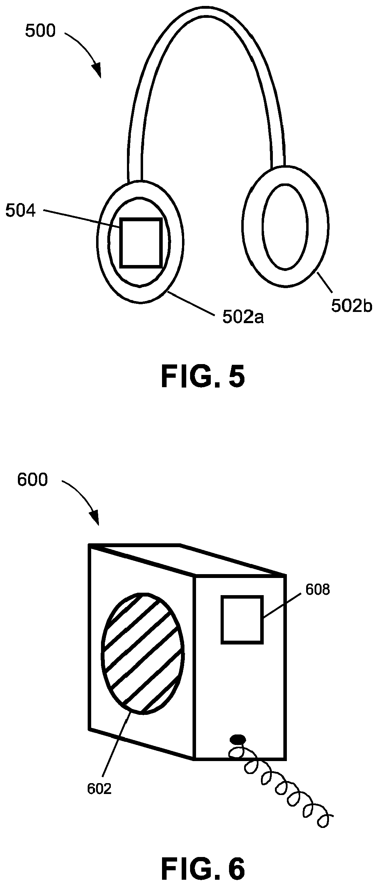

FIG. 5 illustrates example welding headphones 500 that include a noise cancellation audio device 504. The example headphones 500 includes stereo speakers 502a, 502b (e.g., transducers), and may be worn in conjunction with a conventional welding helmet to reduce perceptible welding noise to the wearer of the headphones 500. The noise cancellation audio device 504 generates signal(s) for output by the speakers 502a, 502b based on a received signal representative of the welding current and/or the audible noise generated by the welding arc. The noise cancellation audio device 504 may be implemented using the noise cancellation audio device 400 of FIG. 4. While the example of FIG. 5 is implemented using headphones, in other examples the welding headphones 500 may be implemented using earbuds and/or any other type of personal audio playback devices.

FIG. 6 illustrates an example welding type speaker system 600 that include a noise cancellation audio device 608. The example speaker system 600 includes one or more speakers 602 (e.g., transducers), and may set adjacent to a weld operator and/or adjacent a welding workpiece to reduce perceptible welding noise to the wearer of the speaker system 600. The noise cancellation audio device 608 generates signal(s) for output by the speaker(s) 602 based on a received signal representative of the welding current and/or the audible noise generated by the welding arc. The noise cancellation audio device 608 may be implemented using the noise cancellation audio device 400 of FIG. 4.

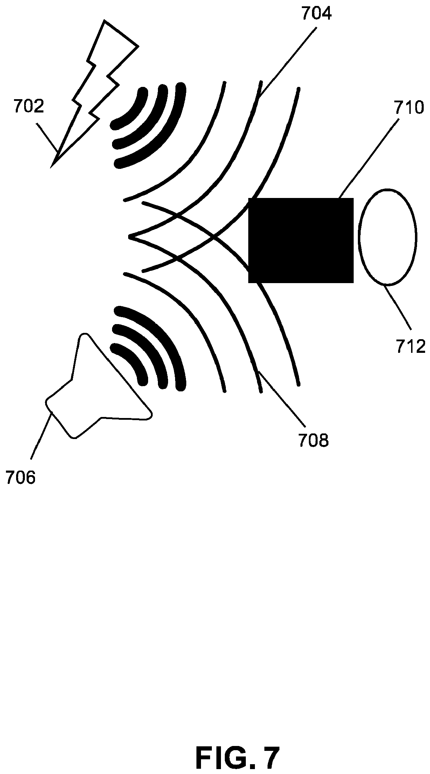

FIG. 7 illustrates an example instance of welding arc noise cancellation by destructive interference of two sound waves. The source of noise may be, for example, an electric arc 702 associated with an AC weld process, or DC pulse process and/or an AC pulse process. Noise sound waves 704 may be emanating from the noise source (e.g., the electric arc 702) and propagate in various directions. In the example of FIG. 7, a noise cancellation audio device 706, such as the example noise cancellation audio devices disclosed herein, generates destructive interference sound waves 708. The noise cancellation audio device 706 generates the destructive interference sound waves 708 to be out of phase by approximately 180 degrees with the noise sound waves 704 (e.g., as close to a 180 degree phase difference as can be achieved by the noise cancellation audio device 706, automatically and/or manually adjusted to be close to a 180 degree phase difference, etc.). As a result, when the noise sound waves 704 combine with the destructive interference sound waves 708, combined resulting acoustic sound waves 710 are received at a receiver 712 (e.g., the weld operator's ear), and will preferably have a lower magnitude than the noise sound waves 704. as a result, the receiver 712 (e.g. weld operator's ear) will perceive a lower magnitude noise or no noise at all. As the phase difference between the noise sound waves 704 and the destructive interference sound waves 708 approaches 180 degrees, the noise that will be perceived by the receiver 712 is further reduced.

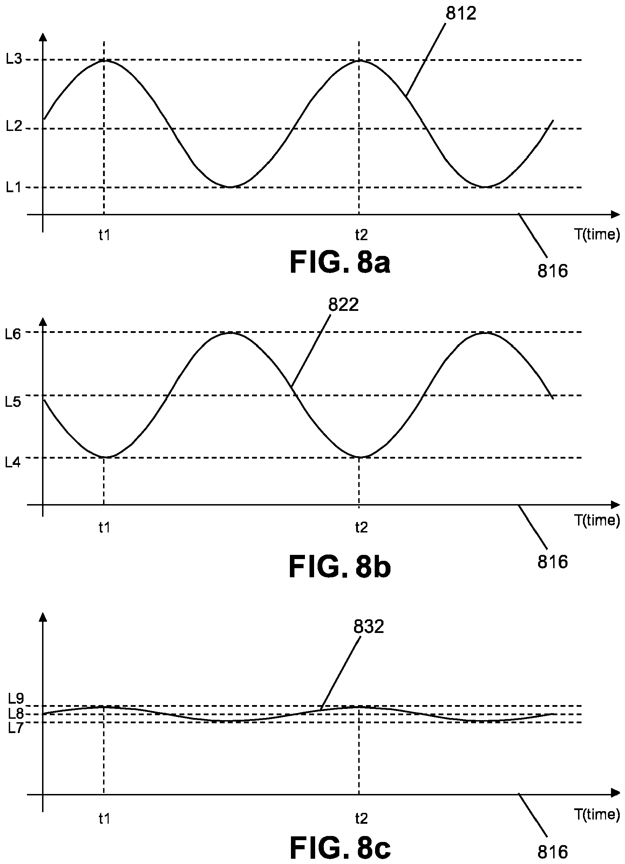

FIG. 8a illustrates an example welding arc noise sound wave 812 with respect to time 816. FIG. 8B illustrates an example destructive interference sound wave 822 with respect to time 816. FIG. 8C illustrates an example resulting acoustic wave 832 with respect to time 816, as perceived by a weld operator (e.g., the weld operator 110 of FIG. 1).

As illustrated in FIG. 8A, the noise sound wave 812 peaks at the peak magnitude L3 at times t1 and t2. The time difference between t1 and t2 is representative of the frequency of the noise sound wave 812 and, therefore, the frequency (or pulses per second) of the welding current waveform. The noise sound wave 812 may be example of the noise sound wave 704 depicted in FIG. 7.

As mentioned above, the example noise cancellation audio device 400 of FIG. 4 generates a destructive interference sound wave, such as the sound wave 822 of FIG. 8B. In particular, the destructive interference sound wave 822 is generated to have the same frequency as the noise sound wave 812, but to have a 180-degree phase shift. To this end, the example destructive interference sound wave 822 is generated to have a lower peak magnitude L4 at times t1 and t2 when the noise sound wave 812 reaches the upper peak magnitude L3. Conversely, the example destructive interference sound wave 822 is generated to have an upper peak magnitude L6 when the noise sound wave 812 reaches the lower peak magnitude LI.

The resulting acoustic wave 832 is a combination of the noise sound wave 812 and the destructive interference sound wave 822. As depicted in FIG. 8C, the resulting acoustic wave 832 has a lower peak magnitude L9 than the peak magnitudes L3 and L6 of the constituent waves 812, 822. While the example upper and lower peak magnitudes are illustrated in FIGS. 8A and 8B as aligned at times t1 and t2, the resulting acoustic wave 832 may have a similarly reduced magnitude even if the magnitudes of the constituent waves 812, 822 are not perfectly matched and/or if the phase difference between the constituent waves 812, 822 is not exactly 180 degrees.

In some examples, the noise cancellation audio device 400 determines (e.g., measures and/or estimates) the times at which predetermined points in the noise sound wave 812 occur. For example, the noise cancellation audio device 400 may determine the times t1 and t2 at which the peak magnitude L3 is reached by the noise sound wave 812. Using the determined times t1 and t2, the noise cancellation audio device 400 synchronizes the destructive interference sound wave 822 by adjusting the phase, frequency, and/or other characteristic(s) of the destructive interference sound wave 822 to match the times at which the destructive interference sound wave 822 is at the lower peak magnitude to the times t1 and t2 at which the noise sound wave 812 is at the upper peak magnitude.

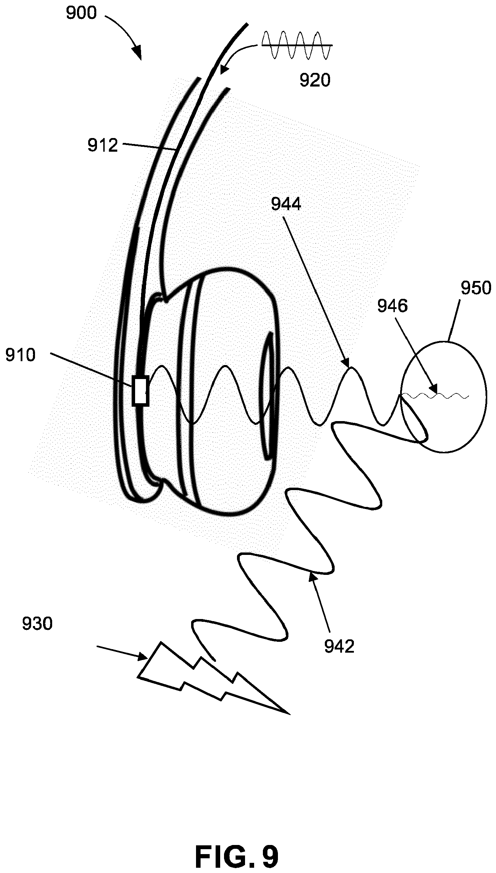

FIG. 9 illustrates an example of operation of the noise cancellation audio device(s) of FIGS. 1, 3, 4, 5, and/or 6. The noise cancellation audio device 910 receives one or more signals 920 representative of a welding current signals (e.g., from a current sensor, from a welding power supply, etc.). The welding current, and the corresponding arc 930, generate welding arc noise sound waves 942 (e.g., the noise sound waves 812 of FIG. 8A) which can ordinarily be perceived by a receiver 950. The signal(s) 920 may be representative of, for example, the current waveform generated by the welding-type power supply 150, 202 of FIGS. 1 and/or 2.

The noise cancellation audio device 910 receives the signal(s) 920 via wires 912, and converts the signal(s) 920 to destructive interference sound waves 944 (e.g., the destructive interference sound wave 822 of FIG. 8B). As mentioned above, the destructive interference sound waves 944 are out of phase with the welding arc noise sound waves by approximately 180 degrees. The noise sound waves 942 combine with the destructive sound waves 944 at the receiver 950 to result in acoustic sound waves 946 (e.g., the resulting acoustic waves 832 of FIG. 8C) that have a magnitude lower than the noise sound waves 942, thereby reducing the perceived noise level of the welding arc 930.

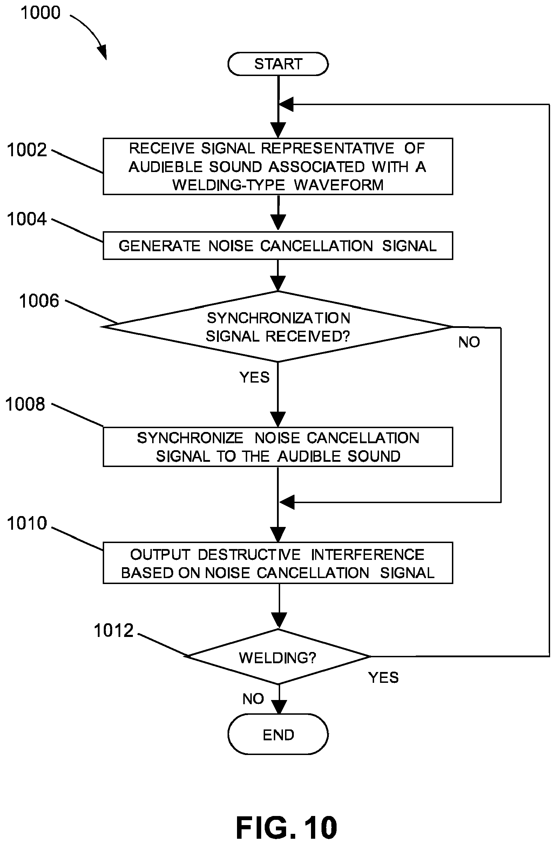

FIG. 10 is a flowchart representative of example machine-readable instructions 1000 which may be executed by disclosed example noise cancellation audio devices, such as the noise cancellation audio devices 112, 320, 400, 500, 600 of FIGS. 1, 3, 4, 5, and/or 6 to generate destructive interference to reduce perceived welding arc noise. The example instructions 1000 are described below with reference to the noise cancellation audio device 400 of FIG. 4. While the instructions 1000 are illustrated sequentially, the noise cancellation audio device 400 may perform multiple ones of the blocks 1002-1014 simultaneously and continuously to generate and output the noise cancellation audio.

At block 1002, the noise cancellation audio device 112 receives (e.g., via the communication circuitry 420, the antenna 422, the network interface 424, and/or the cable connector 426 of FIG. 4) a signal representative of an audible sound associated with a welding-type waveform. For example, the communication circuitry 420 may receive a signal identifying one or more frequencies of the welding-type waveform, magnitude(s) of the welding-type waveform (which may correspond to the one or more frequencies), a current measurement signal, FFT data, an identifier of a characteristic of the welding-type waveform, and/or any other signal representative of the welding-type waveform and/or current. The received signal(s) may be analog or digital.

At block 1004, the noise cancellation audio device 112 generates (e.g., via the audio processing circuitry 440) a noise cancellation signal to reduce the noise generated by the arc. For example, the audio processing circuitry 440 may generate the noise cancellation signal to have a 180-degree phase difference and matching magnitudes to the one or more frequencies in the audible sound represented by the first signal. In some examples, the audio processing circuitry 440 determines the welding noise cancellation signal based on characteristics identified in the received signal using a lookup table and/or a function.

At block 1006, the noise cancellation audio device 112 determines (e.g., via the control circuitry 410 and/or the processor(s) 412) whether a synchronization signal has been received. For example, the noise cancellation audio device 112 may determine whether a synchronization signal has been received via the communication circuitry 420, via the microphone 432, via a current sensor, and/or any other input device.

If a synchronization signal has been received (block 1006), at block 1008 the audio processing circuitry 440 synchronizes the noise cancellation signal to the audible sound based on the synchronization signal. For example, the audio processing circuitry 440 may adjust the phase(s) and/or frequenc(ies) of the noise cancellation signal based on identified peaks and/or other identified points in the audible noise.

After synchronizing the noise cancellation signal (block 1008), or if a synchronization signal has not been received (block 1006), at block 1010 one or more transducers (e.g., the speaker(s) 434) output destructive interference based on the noise cancellation signal. For example, the speaker(s) 434 may output the destructive interference sound waves 944 of FIG. 9.

At block 1012, the control circuitry 410 determines whether the welding process is still in progress. If the welding process is in progress (block 1012), control returns to block 1002 to continue mitigating perceived welding noise. When welding is no longer occurring (block 1012), the example instructions 1000 end.

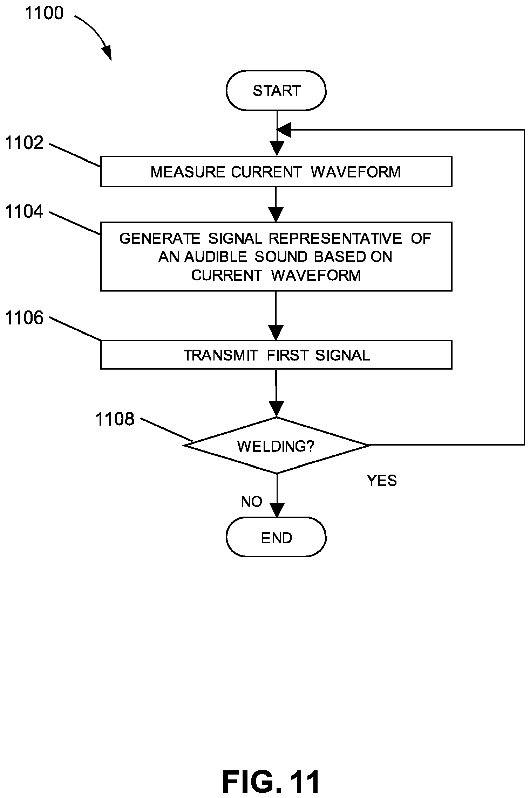

FIG. 11 is a flowchart representative of example machine-readable instructions 1100 which may be executed by the example power supply 150, 202 and/or the example noise cancellation audio device(s) 112, 320, 400, 500, 600 of FIGS. 1, 3, 4, 5, and/or 6 to measure a current of a welding-type waveform. The example instructions 1100 may be performed to provide analog and/or digital data to a noise cancellation audio device about the welding-type waveform and/or the welding-type current, to enable the noise cancellation audio device to generate a destructive interference signal. The example instructions 1100 are described below with reference to the power supply 202 of FIG. 2. While the instructions 1100 are illustrated sequentially, the power supply 202 may perform multiple ones of the blocks 1102-1108 simultaneously and continuously to output the current waveform information.

At block 1102, the current sensor 230 measures a signal indicative of the welding current that is being generated by the power supply 202. For example, the current sensor 230 may measure an output of a current transformer or other sensor.

At block 1104, the sensor 230 generates a signal based on the measured signal. For example, the sensor 230 may generate an analog or digital signal identifying one or more frequencies of the welding-type waveform, magnitude(s) of the welding-type waveform (which may correspond to the one or more frequencies), a current measurement signal, FFT data, an identifier of a characteristic of the welding-type waveform, and/or any other signal representative of the welding-type waveform and/or current.

At block 1106, the communications transceiver 224 transmits the signal. For example, the communication may occur using any wireless or wired media, directly and/or via a communications network.

At block 1108, the control circuitry 210 determines whether the welding process is still in progress. If the welding process is in progress (block 1108), control returns to block 1102 to continue mitigating perceived welding noise. When welding is no longer occurring (block 1108), the example instructions 1100 end.

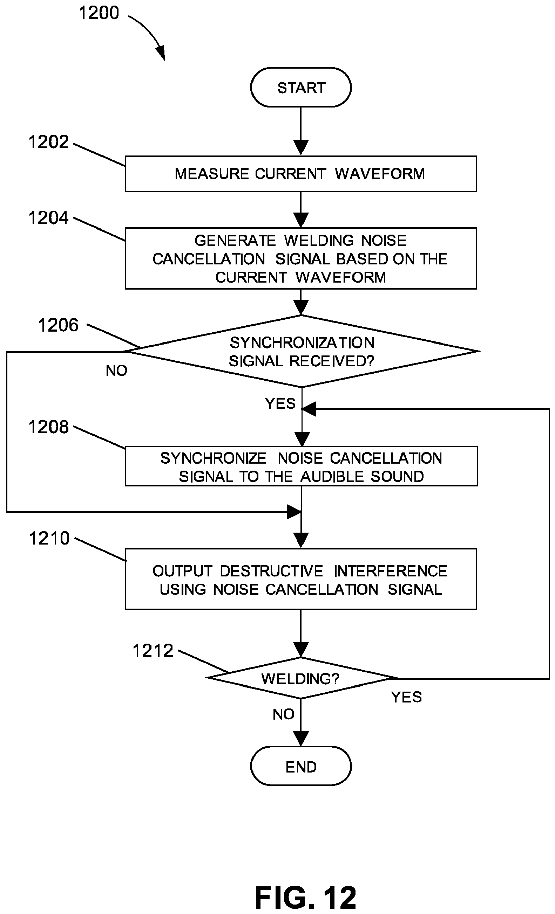

FIG. 12 is a flowchart representative of example machine-readable instructions 1200 which may be executed by the example noise cancellation audio device(s) 112, 320, 400, 500, 600 of FIGS. 1, 3, 4, 5, and/or 6 to generate destructive interference to reduce perceived welding arc noise. The example instructions 1200 are described below with reference to the noise cancellation audio device 400 of FIG. 4. While the instructions 1200 are illustrated sequentially, the noise cancellation audio device 400 may perform multiple ones of the blocks 1202-1014 simultaneously and continuously to generate and output the noise cancellation audio.

At block 1202, the noise cancellation audio device(s) 400 measures a current waveform (e.g., the current in a welding circuit) via a current waveform sensor. For example, the input device(s) 436 may include a current sensor, and/or the cable connector(s) 426 of FIG. 4 may be connected to an external current sensor.

At block 1204, the noise cancellation audio device 112 generates (e.g., via the audio processing circuitry 440) a noise cancellation signal based on the measured current waveform to reduce the noise generated by the arc. For example, the audio processing circuitry 440 may generate the noise cancellation signal to have a 180-degree phase difference and matching magnitudes to the one or more frequencies in the audible sound represented by the current waveform. In some examples, the audio processing circuitry 440 determines the welding noise cancellation signal based on characteristics identified in the measured current waveform using a lookup table and/or a function, by performing an FFT on the measured current waveform, and/or other signal processing.

At block 1206, the noise cancellation audio device 112 determines (e.g., via the control circuitry 410 and/or the processor(s) 412) whether a synchronization signal has been received. For example, the noise cancellation audio device 112 may determine whether a synchronization signal has been received via the microphone 432, via the current sensor, and/or any other input device.

If a synchronization signal has been received (block 1206), at block 1208 the audio processing circuitry 440 synchronizes the noise cancellation signal to the audible sound based on the synchronization signal. For example, the audio processing circuitry 440 may adjust the phase(s) and/or frequenc(ies) of the noise cancellation signal based on identified peaks and/or other identified points in the audible noise.

After synchronizing the noise cancellation signal (block 1208), or if a synchronization signal has not been received (block 1206), at block 1210 the transducer (e.g., the speaker(s) 432 of FIG. 4) outputs destructive interference using the noise cancellation signal to partially or completely cancel the audible noise at the weld operator's ear.

At block 1212, the control circuitry 410 determines whether the welding process is still in progress. If the welding process is in progress (block 1212), control returns to block 1202 to continue mitigating perceived welding noise. When welding is no longer occurring (block 1212), the example instructions 1200 end.

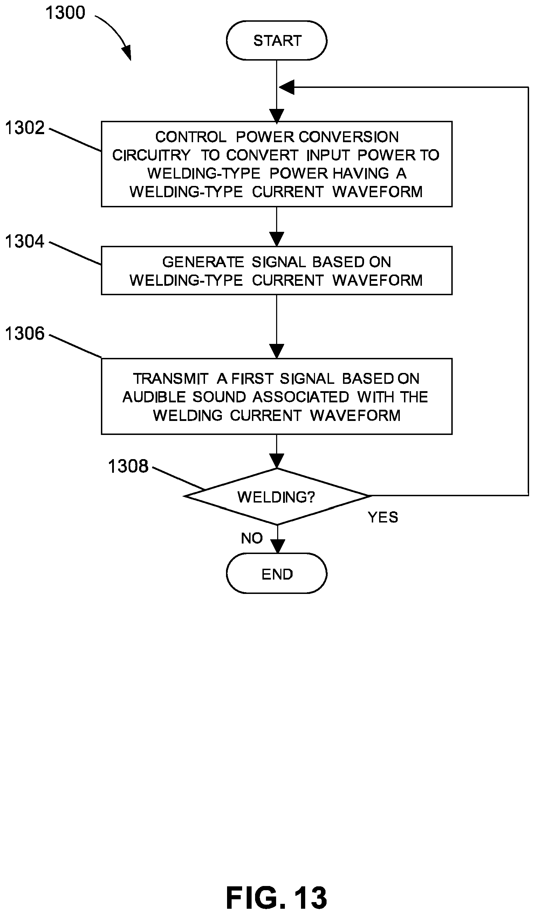

FIG. 13 is a flowchart representative of example machine-readable instructions 1300 which may be executed by the example power supply 150, 202 of FIGS. 1 and/or 2 to transmit a signal based on an audible sound waveform. The example instructions 1300 may be performed to provide analog and/or digital data to a noise cancellation audio device about the welding-type waveform and/or the welding-type current, to enable the noise cancellation audio device to generate a destructive interference signal. The example instructions 1300 are described below with reference to the power supply 202 of FIG. 2. While the instructions 1300 are illustrated sequentially, the power supply 202 may perform multiple ones of the blocks 1102-1108 simultaneously and continuously to output the current waveform information.

At block 1302, the control circuitry 210 controls the power conversion circuitry 232 to convert input power to welding-type power. In the example instructions 1300, the welding-type power has a welding-type current waveform, such as an AC waveform or an AC and/or DC pulse waveform. The control circuitry 210 controls the power conversion circuitry 232 based on parameters that specify the characteristics of the welding-type power and/or the welding-type current waveform, such as frequency (or pulses per second), a target current, a peak current, a background current, an inductance, and/or any other AC and/or pulse parameters.

At block 1304, the control circuitry 210 generates a signal based on the welding-type current waveform. The generated signal is representative of the welding-type current and/or an audible sound resulting from the welding-type current. In some examples, the control circuitry 210 generates the signal based on the parameters used to control the power conversion circuitry 232. Additionally or alternatively, the control circuitry 210 may generate the signal based on a measurement of the output current via the current sensor 230. The signal may be analog or digital, and may include information such as one or more frequencies of the current waveform, one or more magnitudes corresponding to the one or more frequencies, and/or any other information about the current.

In some examples, the control circuitry 210 only includes information about frequencies having at least a threshold magnitude, in which the threshold magnitude corresponds to a threshold volume at which the frequency is considered to be perceptible to the welder. The threshold magnitude or threshold volume may be adjusted based on the individual welder or environment in which the welding process is occurring.

At block 1306, the transmitter circuitry 228 transmits a first signal based on the audible sound associated with the welding current waveform. For example, the transmitter circuitry 228 may transmit the first signal to noise cancellation audio device via a wireless or wired communication, which may be direct or via a communications network.

At block 1308, the control circuitry 210 determines whether the welding process is still in progress. If the welding process is in progress (block 1308), control returns to block 1302 to continue mitigating perceived welding noise. When welding is no longer occurring (block 1308), the example instructions 1300 end.

The present methods and systems may be realized in hardware, software, and/or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application-specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein. As used herein, the term "non-transitory machine-readable medium" is defined to include all types of machine-readable storage media and to exclude propagating signals.

As used herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or." As an example, "x and/or y" means any element of the three-element set {(x), (y), (x, y)}. In other words, "x and/or y" means "one or both of x and y." As another example, "x, y, and/or z" means any element of the seven-element set {(x), (y), (z), (x, y), (x, z), (y, z), (x, y, z)}. In other words, "x, y and/or z" means "one or more of x, y and z." As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present methods and/or system have been described with reference to certain implementations, it will be understood by those skilled in the art that various changes may be made and equivalents may be substituted without departing from the scope of the present method and/or system. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims, both literally and under the doctrine of equivalents.

Certain implementations are described in the following numbered clauses:
Clause 1. An audio device, comprising:
   communication circuitry configured to receive a first signal representative of an audible sound associated with a welding-type waveform;
   audio processing circuitry configured to generate, based on the first signal, a welding noise cancellation signal to produce destructive interference to the audible sound; and
   a transducer configured to output the destructive interference using the welding noise cancellation signal.
Clause 2. The audio device as defined in clause 1, wherein the communication circuitry is configured to receive a synchronization signal, and the audio processing circuitry is configured to synchronize the noise cancellation signal based on the synchronization signal.
Clause 3. The audio device as defined in clause 1, wherein the first signal identifies a frequency of the welding-type waveform.
Clause 4. The audio device as defined in clause 1, wherein the first signal identifies a magnitude of the welding-type waveform.
Clause 5. The audio device as defined in clause 1, further comprising a microphone configured to receive the audible sound, the audio processing circuitry configured to synchronize the welding noise cancellation signal based on the audible sound.
Clause 6. The audio device as defined in clause 1, wherein the audio device comprises at least one of headphones, a welding helmet, or a loudspeaker.
Clause 7. The audio device as defined in clause 1, wherein the first signal is an analog signal representative of the welding-type waveform.
Clause 8. The audio device as defined in clause 1, wherein the first signal comprises an identifier of a characteristic of the welding-type waveform, and the audio processing circuitry is configured to determine the welding noise cancellation signal based on the characteristic.
Clause 9. The audio device as defined in clause 8, wherein the audio processing circuitry is configured to determine the welding noise cancellation signal based on the characteristic using at least one of a lookup table or a function.
Clause 10. The audio device as defined in clause 1, wherein the welding-type waveform comprises at least one of an AC waveform or a DC pulse waveform.
Clause 11. A welding apparatus, comprising:
   a current sensor configured to measure a current of a welding-type waveform; and
   a transmitter configured to transmit a first signal representative of an audible sound associated with the welding-type waveform.
Clause 12. The welding apparatus as defined in clause 11, wherein the first signal identifies at least one of a frequency of the welding-type waveform or a magnitude of the welding-type waveform.
Clause 13. The welding apparatus as defined in clause 11, wherein the first signal comprises an analog waveform based on the measurement by the current sensor.
Clause 14. The welding apparatus as defined in clause 11, wherein the current sensor comprises at least one of a current transformer, Hall Effect sensor, a shunt current sensor, or a Rogowski coil configured to be coupled to a welding circuit transmitting the welding-type waveform.
Clause 15. A welding apparatus, comprising:
   a current sensor configured to measure a current of a welding-type waveform;
   audio processing circuitry configured to generate, based on the current, a welding noise cancellation signal to produce destructive interference to the audible sound; and
   a transducer configured to output the destructive interference using the welding noise cancellation signal.
Clause 16. A welding-type power supply, comprising:
   power conversion circuitry configured to convert input power to welding-type power having a welding-type waveform; and
   a transmitter configured to transmit a first signal based on an audible sound associated with the welding-type waveform.
Clause 17. The welding-type power supply as defined in clause 16, further comprising control circuitry configured to:
   control the power conversion circuitry to output the welding-type power; and
   generate the first signal based on the welding-type waveform.
Clause 18. The welding-type power supply as defined in clause 16, wherein the first signal identifies at least one of a frequency of the welding-type waveform or a magnitude of the welding-type waveform.
Clause 19. The welding-type power supply as defined in clause 16, wherein the first signal comprises an analog waveform representative of the welding-type waveform.
Clause 20. The welding-type power supply as defined in clause 16, wherein the first signal comprises an audio signal representative of destructive interference to the audible sound associated with the welding-type waveform.

## Claims

1. An audio device, comprising:
communication circuitry configured to receive a first signal representative of an audible sound associated with a welding-type waveform;
audio processing circuitry configured to generate, based on the first signal, a welding noise cancellation signal to produce destructive interference to the audible sound; and
a transducer configured to output the destructive interference using the welding noise cancellation signal.

2. The audio device as defined in claim 1, wherein the communication circuitry is configured to receive a synchronization signal, and the audio processing circuitry is configured to synchronize the noise cancellation signal based on the synchronization signal.

3. The audio device as defined in claim 1, further comprising a microphone configured to receive the audible sound, the audio processing circuitry configured to synchronize the welding noise cancellation signal based on the audible sound.

4. The audio device as defined in claim 1, wherein the audio device comprises at least one of headphones, a welding helmet, or a loudspeaker.

5. The audio device as defined in claim 1, wherein the first signal is an analog signal representative of the welding-type waveform.

6. The audio device as defined in claim 1, wherein the first signal comprises an identifier of a characteristic of the welding-type waveform, and the audio processing circuitry is configured to determine the welding noise cancellation signal based on the characteristic, and optionally wherein the audio processing circuitry is configured to determine the welding noise cancellation signal based on the characteristic using at least one of a lookup table or a function.

7. The audio device as defined in claim 1, wherein the welding-type waveform comprises at least one of an AC waveform or a DC pulse waveform.

8. A welding apparatus, comprising:
a current sensor configured to measure a current of a welding-type waveform; and
a transmitter configured to transmit a first signal representative of an audible sound associated with the welding-type waveform.

9. The welding apparatus as defined in claim 8, wherein the first signal comprises an analog waveform based on the measurement by the current sensor.

10. The welding apparatus as defined in claim 8, wherein the current sensor comprises at least one of a current transformer, Hall Effect sensor, a shunt current sensor, or a Rogowski coil configured to be coupled to a welding circuit transmitting the welding-type waveform.

11. A welding apparatus, comprising:
a current sensor configured to measure a current of a welding-type waveform;
audio processing circuitry configured to generate, based on the current, a welding noise cancellation signal to produce destructive interference to the audible sound; and
a transducer configured to output the destructive interference using the welding noise cancellation signal.

12. A welding-type power supply, comprising:
power conversion circuitry configured to convert input power to welding-type power having a welding-type waveform; and
a transmitter configured to transmit a first signal based on an audible sound associated with the welding-type waveform.

13. The welding-type power supply as defined in claim 12, further comprising control circuitry configured to:
control the power conversion circuitry to output the welding-type power; and
generate the first signal based on the welding-type waveform.

14. The audio device as defined in claim 1, the welding apparatus as defined in claim 8, or the welding-type power supply as defined in claim 12, wherein the first signal identifies at least one of a frequency of the welding-type waveform or a magnitude of the welding-type waveform.

15. The welding-type power supply as defined in claim 12, wherein the first signal comprises:
an analog waveform representative of the welding-type waveform; or
an audio signal representative of destructive interference to the audible sound associated with the welding-type waveform.
